# EUROPÄISCHE PATENTANMELDUNG

(11) **EP 0 745 842 A1**
(43) Veröffentlichungstag der Anmeldung: **04.12.1996**
(21) Anmeldenummer: 95108503.4
(22) Anmeldetag: 02.06.1995
(51) Int. Cl.: G01N 27/12

(54) **Feuchtefühler**

(71) Anmelder: STAEFA CONTROL SYSTEM SCS AG, CH-8712 Stäfa (CH)
(72) Erfinder: Waldburger, Daniel, CH-8645 Jona (CH); Muggli, Jürg, Dr., CH-8708 Männedorf (CH)
(74) Vertreter: Dittrich, Horst, Dr.

(57) **Zusammenfassung**

Der Feuchtefühler (1) besteht aus einem Sensorhalter (2), aus einem Sensor (3) und aus einer Abschirmung (7) zum Schutz des Sensors (3) gegen Verschmutzung. Der Sensor (3) und die Abschirmung (7) sind im Sensorhalter (2) versenkt angeordnet, und die Abschirmung (7) ist durch ein Feinstfilter mit einer Porengrösse < 1µm gebildet.

## Beschreibung

Die vorliegende Erfindung betrifft einen Feuchtefühler zur Erfassung der Feuchtigkeit in einem Raum, mit einem Sensorhalter, mit einem an diesem angeordneten Sensor und mit einer Abschirmung zum Schutz des Sensors gegen Verschmutzung.

Derartige Feuchtefühler gelangen vor allem in der Heizung, Lüftung und Klimatechnik und in verwandten Gebieten zum Einsatz und dienen dazu, die Feuchte in einem Raum oder in einem Lüftungskanal zu erfassen und in ein normiertes Schnittstellensignal umzuwandeln. Das Schnittstellensignal wird als Führungsgrösse für einen die jeweilige Befeuchtungsstrecke regelnden Regler benutzt. Bei Anordnung in einem Lüftungskanal sind die Feuchtefühler in der Regel stab- oder rohrförmig ausgebildet und ragen quer in den Kanal; der Sensor ist im Bereich des in den Kanal ragenden Endes des Fühlers angeordnet. Bei bekannten Feuchtefühlern dieser Art ist das den Sensor tragende Ende des Fühlers käfigartig ausgebildet, wobei der Sensor im Inneren des durch Öffnungen in der Wand des Fühlers gebildeten Käfigs angeordnet und dem direkten Luftstrom ausgesetzt ist. Die Abschirmung ist durch ein relativ grobmaschiges Filter mit einer Porengrösse zwischen etwa 5 und 15µm gebildet. Das hat zur Folge, dass der Sensor wegen des direkten Kontakts mit dem Luftstrom nur eine geringe Lebensdauer aufweist, und dass das ebenfalls im direkten Luftstrom liegende Filter rasch verschmutzt und dewegen relativ häufig ausgewechselt werden muss.

Durch die Erfindung sollen nun diese bekannten Feuchtefühler hinsichtlich Lebensdauer des Sensors und Verschmutzung des Filters wesentlich verbessert und der entsprechende Wartungsaufwand soll merklich reduziert werden.

Diese Aufgabe wird erfindungsgemäss dadurch gelöst, dass der Sensor und die Abschirmung im Sensorhalter versenkt angeordnet sind, und dass die Abschirmung durch ein Feinstfilter mit einer Porengrösse < 1µm gebildet ist.

Durch diese versenkte Montage liegen Sensor und Abschirmung nicht im direkten Luftstrom, was einerseits die Lebensdauer des Sensors wesentlich erhöht und andererseits die Verschmutzung des Filters auf ein Minimum reduziert. Letzteres erhöht die Wartungsintervalle ganz beträchtlich oder macht unter Umständen sogar eine Wartung des Filters unnötig. Die Ausbildung der Abschirmung als Feinstfilter hat den weiteren Vorteil, dass die Messung der Feuchte nach dem Diffusionsprinzip erfolgt und damit von der Luftgeschwindigkeit im Raum oder im Lüftungskanal unabhängig ist.

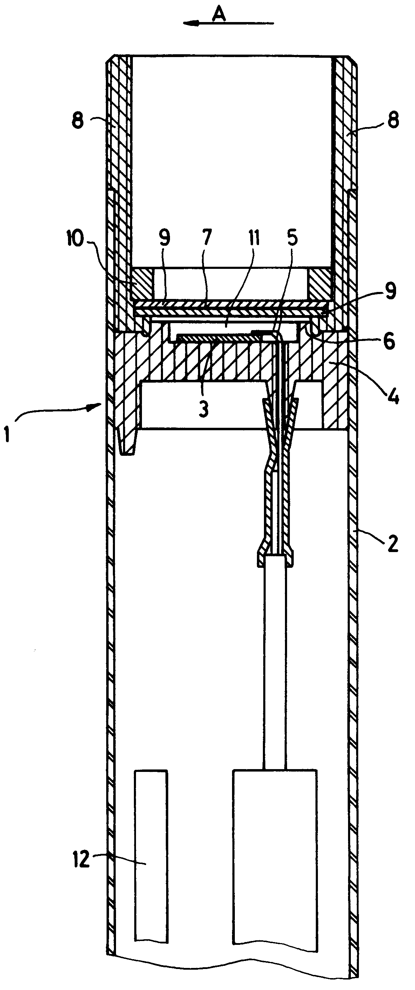

Nachstehend wird die Erfindung anhand eines in der einzigen Figur dargestellten Ausführungsbeispiels näher erläutert, wobei die Figur einen Achsialschnitt durch das in einen Lüftungskanal ragende vordere Ende eines erfindungsgemässen Feuchtefühlers in einem stark vergrösserten Massstab zeigt.

Der mit dem Bezugszeichen 1 bezeichnete Feuchtefühler besteht aus einem Tragrohr 2, das an seinem einen Ende an einer Seitenwand eines Lüftungskanals befestigt ist und quer in den Lüftungskanal ragt, und das an seinem anderen, freien Ende einen Feuchtesensor 3 trägt. Die Längsrichtung des Lüftungskanals und die Richtung des Luftstroms in diesem sind durch einen Pfeil A angedeutet. Darstellungsgemäss ist in das freie Ende des Tragrohres 2 eine runde Platte 4 eingesetzt, die aus einem geeigneten Kunststoff besteht und als Auflage für den Sensor 3 dient. Der Sensor 3 ist beispielsweise ein kapazitiver Feuchtigkeitsgeber der in der CH-A-559 365 beschriebenen Art, mit einem Dielektrikum aus einem Polymerfilm, dessen Dielektrizitätskonstante eine Funktion der von ihm absorbierten Wassermenge ist. Am Sensor 3 sind Kontakte vorgesehen, an denen Kontaktleitungen 5 befestigt sind, zwischen denen die Kapazität gemessen wird. Die Kontaktleitungen 5 sind durch eine Öffnung im Boden der Platte 4 zu einer Auswerteelektronik (nicht dargestellt) geführt.

Die Platte 4 ist an ihrer den Sensor 3 tragenden Seite mit einem ringförmigen Steg 6 versehen, der den Sensor 3 seitlich umschliesst. Gegen das freie Ende des Tragrohres 2 hin ist vor dem Sensor 3 ein diesen gegen Verschmutzung schützendes Filter 7 angeordnet. Dieses ist in einem büchsenartigen, in das Tragrohr 2 eingesetzten, beispielsweise in dieses hineingeschobenen oder hineingedrückten, Filterhalter 8 gehalten. Letzterer weist an seinem der Platte 4 zugewandten Ende einen Boden mit einer Bohrung auf, wodurch an dem genannten Ende des Filterhalters 8 zwischen dessen Innenwand und der Bohrung eine Abstufung gebildet ist. Der Durchmesser der Bohrung ist etwas grösser als der Aussendurchmesser des ringförmigen Stegs 6, so dass die genannte Abstufung bei eingesetztem Filterhalter 8 den Steg 6 aussen umfasst. In die Abstufung ist das Filter 7 eingelegt, zu dessen beiden Seiten je ein Stützsieb 9 angeordnet ist; Filter 7 und Stützsiebe 9 sind im Filterhalter 8 durch einen Anpressring 10 fixiert.

Der Filterhalter 8 liegt mit seinem Boden an der Platte 4 an und die Höhe des Stegs 6 und die Dicke des Bodens des Filterhalters 8 sind so gewählt, dass zwischen der Stirnfläche des Stegs 6 und dem benachbarten Stützsieb 9 ein schmaler Spalt gebildet ist. Somit besteht zwischen Sensor 3 und Filter 7 ein definierter Abstand. Der Filterhalter 8 ist vorzugsweise so ausgebildet, dass das Einsetzen in und insbesondere das Herausziehen aus dem Tragrohr 2 möglichst einfach erfolgen kann. Zu diesem Zweck kann beispielsweise die Wand des Filterhalters 8 im Abstand vom Anpressring 10 mit Bohrungen versehen sein, in die eine Griffstange eingeschoben werden kann.

Der Filterhalter 8 besteht aus dem gleichen Kunststoff wie die Platte 4, vorzugsweise aus einem thermoplastischen Kautschuk, wie beispielsweise der von der Firma Monsanto unter dem Warenzeichen "Santoprene" vertriebene Kautschuk. Der Anpressring 10 besteht beispielsweise aus Aluminium, und das Filter 7 ist ein sogenanntes Feinstfilter mit einer Porengrösse < 1µm, vorzugsweise < 0.5µm, das aus regenerierter Zellulose besteht. Bei einer bevorzugten Ausführungsform beträgt die Porengrösse 0.45 µm. Da dieses Filter nicht selbsttragend ist, sind die beiden Stützsiebe 9 vorgesehen, die aus Polypropylen bestehen. Die Verwendung des Feinstfilters 7 ermöglicht zusammen mit der versenkten Montage von Filter 7 und Sensor 3 eine Messung nach dem Diffusionsprinzip, die unabhängig von der Luftgeschwindigkeit im Lüftungskanal ist.

Die wesentlichsten Anforderungen an das Filter 7 und dessen Anordnung sind einerseits Schutz des Sensors 3 vor Verschmutzung und andererseits Sicherstellung einer kurzen Ansprechzeit, letzteres trotz der Messung nach dem Diffusionsprinzip. Eine Verschmutzung des Sensors muss unbedingt vermieden werden, weil der Schmutz, der meistens ionischen Charakter hat, durch Auflösung der Ionen die Messung drastisch verfälscht und durch in den Sensor diffundierende Ionen in diesem einen Kurzschluss hervorrufen kann. Zur Vermeidung von Verschmutzung sollte das Filter 7 möglichst feinmaschig sein. Ist es aber zu dicht, dann geht der Austausch mit der Luft sehr langsam vor sich und die Ansprechzeit wird zu lang.

Die Ansprechzeit des Fühlers 1 wird nicht nur durch die Porengrösse des Filters 7 beeinflusst, sondern auch durch das Volumen 11 zwischen Sensor 3 und Filter 7. Da dieses Volumen bei sonst gleichen Parametern durch den Abstand zwischen Sensor 3 und Filter 7 bestimmt wird, kommt diesem Abstand eine erhebliche Bedeutung zu. Wegen der geringen Porengrösse von < 0.5 µm sollte der genannte Abstand möglichst klein sein, ohne dass es aber zu einem Kontakt zwischen Sensor und Filter kommt. Dieses Ziel wird dadurch erreicht, dass das Filter 7 in die im Bereich des Bodens des Filterhalters 8 gebildete Abstufung eingelegt und dass die Dicke des Bodens des Filterhalters 8 so gewählt ist, dass Sensor 3 und Filter 7 einander gerade nicht berühren.

Der Feuchtefühler 1 kann zusätzlich zum Sensor 3 für die Feuchtigkeit noch einen Temperaturfühler 12 enthalten, der an der Innenwand des Tragrohrs 2 angeordnet und ebenso wie der Sensor 3 mit der Auswertelektronik des Feuchtefühlers 1 verbunden ist. Der Temperaturfühler 12 ist vorzugsweise durch einen PTC-Widerstand gebildet. Zur Sicherstellung eines möglichst raschen Temperaturübergangs vom Tragrohr 2 zum Temperaturfühler 12 ist der letzere durch ein gut wärmeleitendes Organ, vorzugsweise durch eine Metallfeder, an die Wand des Tragrohrs 2 angepresst.

Der beschriebene Feuchtefühler weist gegenüber bekannten Feuchtefühlern die folgenden vorteilhaften Eigenschaften auf:
- Der Sensor ist durch versenkte Montage im Tragrohr vor dem Luftstrom geschützt, wodurch seine Lebensdauer wesentlich erhöht wird.
- Durch die Verwendung eines Feinstfilters mit einer Porengrösse < 0.5 µm kann die Messung der Feuchtigkeit nach dem Diffusionsprinzip erfolgen.
- Durch die Messung nach dem Diffusionsprinzip ist jede Abhängigkeit des Messergebnisses von der Luftgeschwindigkeit ausgeschaltet.
- Dadurch, dass das Filter nicht direkt im Luftstrom liegt, wird die Filterverschmutzung auf ein Minimum reduziert und der Wartungsaufwand drastisch vermindert.
- Durch ein minimales Volumen zwischen Filter und Sensor kann trotz Messung nach dem Diffusionsprinzip eine kurze Ansprechzeit des Fühlers realisiert werden.

Der beschriebene Feuchtefühler ist nicht nur zur Verwendung in Lüftungskanälen geeignet, sondern kann ohne wesentliche Änderungen in jedem beliebigen Raum eingesetzt werden.

## Patentansprüche

1. Feuchtefühler zur Erfassung der Feuchtigkeit in einem Raum, mit einem Sensorhalter, mit einem an diesem angeordneten Sensor und mit einer Abschirmung zum Schutz des Sensors gegen Verschmutzung, dadurch gekennzeichnet, dass der Sensor (3) und die Abschirmung (7) im Sensorhalter (2) versenkt angeordnet sind, und dass die Abschirmung durch ein Feinstfilter mit einer Porengrösse < 1µm gebildet ist.

2. Feuchtefühler nach Anspruch 1, dadurch gekennzeichnet, dass die Porengrösse des Filters (7) kleiner als 0.5µm ist und vorzugsweise 0.45µm beträgt.

3. Feuchtefühler nach Anspruch 1 oder 2, dadurch gekennzeichnet, dass das Filter (7) aus regenerierter Zellulose besteht.

4. Feuchtefühler nach einem der Ansprüche 1 bis 3, dadurch gekennzeichnet, dass zwischen Sensor (3) und Filter (7) ein minimaler gegenseitiger Abstand besteht.

5. Feuchtefühler nach Anspruch 4, dadurch gekennzeichnet, dass der Sensorhalter (2) rohrförmig ausgebildet ist und an seinem in den Raum ragenden freien Ende eine versenkt angeordnete Montageplatte (4) für den Sensor (3) trägt, und dass das Filter (7) in einem am freien Ende des Sensorhalters befestigbaren Filterhalter (8) montiert ist.

6. Feuchtefühler nach Anspruch 5, dadurch gekennzeichnet, dass der Filterhalter (8) in das freie Ende des Sensorhalters (2) einsetzbar ist und an seinem dem Sensor (3) zugewandten Ende eine fensterartige Öffnung aufweist, in der das Filter (7) befestigt ist.

7. Feuchtefühler nach Anspruch 6, dadurch gekennzeichnet, dass das Filter (7) an der genannten fensterartigen Öffnung festgeklemmt und zu beiden Seiten durch je ein Stützsieb (9) abgedeckt ist.

8. Feuchtefühler nach Anspruch 7, dadurch gekennzeichnet, dass die Montageplatte (4) einen gegen das freie Ende des Sensorhalters (2) ragenden ringförmigen Steg (6) aufweist, welcher einen zur Aufnahme des Sensors vorgesehenen Raum umschliesst.

9. Feuchtefühler nach Anspruch 8, dadurch gekennzeichnet, dass der Filterhalter (8) an seinem das Filter (7) tragenden Ende an der Montageplatte (4) anliegt, und dass die fensterartige Öffnung so ausgebildet ist, dass zwischen Sensor (3) und Filter (7) ein Abstand besteht.

10. Feuchtefühler nach Anspruch 9, dadurch gekennzeichnet, dass der Abstand so gewählt ist, dass Filter (7) und Sensor (3) einander gerade nicht berühren.
